(19)

Europäisches
Patentamt
European
Patent Office
Office européen
des brevets

(11) **EP 1 852 417 A1**

(12) **EUROPEAN PATENT APPLICATION**
published in accordance with Art. 158(3) EPC

(43) Date of publication:
**07.11.2007 Bulletin 2007/45**

(21) Application number: **06714674.6**

(22) Date of filing: **21.02.2006**

(51) Int Cl.:
***C07C 403/24*** (2006.01)     ***A23L 1/27*** (2006.01)

(86) International application number:
**PCT/JP2006/303537**

(87) International publication number:
**WO 2006/090871 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.02.2005 JP 2005051689**

(71) Applicant: **Yamaha Hatsudoki Kabushiki Kaisha Iwata-shi, Shizuoka 438-8501 (JP)**

(72) Inventors:
• **ISHIKURA, Masaharu**
  **ai, Iwata-shi, Shizuoka, 4388501 (JP)**

• **OKADA, Yumika**
  **ai, Iwata-shi, Shizuoka, 4388501 (JP)**
• **MURAKAMI, Nagisa**
  **ai, Iwata-shi, Shizuoka, 4388501 (JP)**

(74) Representative: **Schlich, George William et al**
  **Schlich & Co**
  **34 New Road**
  **Littlehampton**
  **West Sussex BN17 5AT (GB)**

(54) **METHOD OF RECOVERING XANTHOPHYLL**

(57)     The present invention provides a method for collecting xanthophylls, the method comprising an adsorption step of contacting a sample containing xanthophylls with a hydrophilic resin to allow the resin to adsorb the xanthophylls; and a desorption step of eluting the xanthophylls adsorbed to the resin with a polar solvent. Preferably, the method comprises a step of washing the resin between the adsorption step and the desorption step. As the resin, a hydrophilic, acrylic and/or methacrylic resin is preferably used.

EP 1 852 417 A1

**Description**

Technical Field

The present invention relates to a method for collecting xanthophyll from an oily material containing xanthophyll. Furthermore, the present invention relates to a xanthophyll-containing product containing xanthophyll at a high concentration.

Background Art

Carotenoids are yellow to red pigments occurring widely in animals and plants. It is known that carotenoids, which have structures based on lycopene, have 500 or more variations, which result from, for example, cyclization at one end of the lycopene molecule and introduction of an oxygen atom into the ring. A hydrocarbon with a cyclized end is called "carotene", and a compound containing an oxygen atom in this carotene structure is called "xanthophyll".

As described above, xanthophyll is a carotene derivative in which an oxygen functional group is introduced into a carotenoid, which is a polyene pigment, and canthaxanthin, zeaxanthin, lutein, adonirubin, β-cryptoxanthin, astaxanthin, and the like are known as xanthophyll.

Xanthophyll is known to be useful as a pigment in food. Moreover, xanthophyll includes numerous compounds having biological activities. For example, C. Sato, New Food Industry, vol. 44, No. 10, pp. 1 to 5, 2002 describes that lutein and zeaxanthin are effective in preventing age-related macular degeneration (AMD). Moreover, K. Yazawa, New Food Industry, vol. 46, No. 5, pp. 17-24, 2004 describes that astaxanthin has an antioxidative effect as a singlet oxygen scavenger or a free radical scavenger and has the effect of suppressing oxidation of LDL (low density lipoprotein) cholesterol, which is a factor that promotes arteriosclerosis, and other effects.

Xanthophyll, which has such excellent beneficial effects, is found in many organisms such as plants, animals, and microorganisms. The concentration of xanthophyll in these organisms is not always high, thus only limited organisms can be used to produce a sufficient quantity of xanthophyll at present. For example, lutein and zeaxanthin are produced by extraction from a fruit or the petals of marigold flowers. Astaxanthin is produced from, for example, microalgae such as green algae and diatoms (e.g., algae from the genus *Haematococcus* and the genus *Dunaliella*) and yeast (e.g., the genus *Phaffia*), although in some cases astaxanthin can be chemically synthesized.

Some xanthophylls have a hydroxyl group. For example, astaxanthin, which is a xanthophyll produced in algae and the like, has two hydroxyl groups. Thus, astaxanthin often is present in the form of a monoester or diester in which a fatty acid is bonded to a hydroxyl group or each of the hydroxyl groups. Especially, the monoester form is present in large quantities, and the free form in which the hydroxyl groups are not bonded to a fatty acid is present in very small trace quantities. Such ester forms generally have higher stability to light, oxygen, and the like than the free form (Masayoshi Kazama et al., The Food Industry, vol. 46, No. 24, pp. 25-35, 2003), and are easy to handle in production, and also are sometimes readily absorbed into the animal body (Phylips E. Browen et al., J. Nutr., vol. 132, pp. 3668-3673, 2002).

However, fatty acid esters of xanthophyll are similar to triglycerides and diglycerides, which are neutral lipids having an ester bond to a fatty acid, in various chemical behaviors. Furthermore, the algae that produce xanthophyll such as astaxanthin also produce the above-mentioned neutral lipids in large quantities. Thus, if xanthophyll is extracted with a common organic solvent, then neutral lipids are extracted together with xanthophyll in a greater quantity than the xanthophyll. That is, neutral lipids containing xanthophyll at a low concentration (10% or less) are collected. Therefore, in order to obtain a high concentration of astaxanthin, it is necessary to separate the fatty acid esters of xanthophyll from the neutral lipids, which are similar to each other in chemical behaviors.

An example of the method for collecting xanthophyll from large quantities of fats and oils (neutral lipids) includes a method based on crystallization. For example, Japanese Patent No. 3266719 discloses a method for purifying astaxanthin by crystallizing astaxanthin from a solution of chloroform containing astaxanthin. Moreover, Japanese Laid-Open Patent Publication (PCT International Application) No. 11-508603 discloses a method for purifying a xanthophyll diester by crystallization from an oil-containing component extracted from the petals of marigold flowers.

However, crystallization using chloroform is not suitable for use in processes for producing food products because of the toxicity of chloroform and other problems. Crystallization has problems such as: the concentration of a substance that is desired to be crystallized is required to be higher than or equal to a predetermined concentration; substances other than the desired substance may be crystallized out; and the purity of the crystallized product decreases when the desired substance does not crystallize readily.

Furthermore, Japanese Laid-Open Patent Publication No. 2004-41147 discloses a method for obtaining an astaxanthin concentrate by supercritical extraction. However, since this method is performed under high-pressure conditions, the equipment is expensive, and furthermore, there are operational problems in that, for example, it is necessary to break up the cell walls of algae, knead the algae with an auxiliary solvent, and form a shaped product.

On the other hand, in order to remove carotenoids which, unlike xanthophylls, have no oxygen atom (e.g.,

carotene) from fats and oils, various methods using a resin have been examined. For example, Japanese Laid-Open Patent Publication No. 61-12657; B. S. Baharin et al., JAOCS, vol. 75, No. 3, pp. 399-404, 1998; R. A. Latip et al., JAOCS, vol. 77, No. 12, pp. 1277-1281, 2000; and K. W. Chan et al., J. Food Lipids, vol. 7, pp. 127-141, 2000 disclose a method for collecting carotene by contacting a carotene-containing crude palm oil (CPO), directly or after having been dissolved in an alcohol, with a synthetic resin made from a styrene-divinylbenzene copolymer to allow the resin to adsorb the carotene, washing the resin with an alcohol, and then eluting the carotene with hexane.

Moreover, Japanese Laid-Open Patent Publication No. 63-5073 describes a method for collecting carotene by dissolving, if necessary, a fat or oil containing carotene in an alcohol, contacting the oil or fat with a polyacrylic ester resin or a polymethacrylic resin, washing the resin with an alcohol, and then eluting carotene with a hydrophobic solvent such as hexane.

In both of the above-described methods for collecting carotene, a very low concentration (about several hundred ppm) of carotene contained in a palm oil is brought into contact with the above-described resins under heating conditions (e.g., 40 to 80°C), so that the necessity for large-scale equipment and other problems occur. Furthermore, no instance in which these resins are used to concentrate xanthophyll is known.

Disclosure of Invention

The present invention provides a method for collecting xanthophylls. In particular, it is an object of the present invention to provide an efficient method for concentrating xanthophylls that are present in an oily material such as neutral lipids, for example, in an extract from algae.

The present invention provides a method for collecting xanthophylls, the method comprising:

an adsorption step of contacting a sample containing the xanthophylls with a hydrophilic resin to allow the hydrophilic resin to adsorb the xanthophylls; and
a desorption step of eluting the xanthophylls adsorbed to the hydrophilic resin with a polar solvent.

In an embodiment, the method further comprises a step of washing the hydrophilic resin between the adsorption step and the desorption step.

In another embodiment, the hydrophilic resin is an acrylic resin.

In a further embodiment, the sample containing xanthophylls that is brought into contact with the hydrophilic resin is an oily material containing xanthophylls.

In a further embodiment, the oily material containing xanthophylls is obtained from an alga.

In a preferred embodiment, the alga is a green alga belonging to the genus *Haematococcus.*

In a further other embodiment, the sample containing xanthophylls that is brought into contact with the hydrophilic resin is dissolved in at least one solvent selected from the group consisting of alcohols, saturated hydrocarbons, aromatic hydrocarbons, ethers, esters, halogenated hydrocarbons, and ketones.

In another embodiment, the alcohols, saturated hydrocarbons, aromatic hydrocarbons, ethers, esters, halogenated hydrocarbons, and ketones are at least one solvent selected from the group consisting of methanol, ethanol, propanol, isopropanol, pentane, hexane, heptane, toluene, xylene, tetrahydrofuran, diethyl ether, methyl acetate, ethyl acetate, dichloromethane, carbon tetrachloride, acetone, and methyl ethyl ketone.

In a further other embodiment, the polar solvent used to elute the xanthophylls is at least one solvent selected from the group consisting of methanol, ethanol, propanol, isopropanol, n-butanol, tetrahydrofuran, diethyl ether, methyl acetate, ethyl acetate, acetone, and methyl ethyl ketone.

In another embodiment, the xanthophylls are astaxanthins.

The present invention also provides a concentrate of xanthophylls, wherein the xanthophylls contain a xanthophyll fatty acid ester as a main component and has a xanthophyll content of 30 wt% or more in terms of free xanthophyll.

In an embodiment, the concentrate is obtained by a method comprising:

contacting a sample containing xanthophylls with a hydrophilic resin to allow the hydrophilic resin to adsorb the xanthophylls; and
eluting the xanthophylls adsorbed to the hydrophilic resin with a polar solvent.

In another embodiment, the concentrate of xanthophylls is a concentrate of astaxanthins.

In a further other embodiment, the astaxanthins are obtained from a green alga belonging to the genus *Haematococcus.*

According to the method of the present invention, a high concentration of xanthophylls is collected by allowing a hydrophilic resin, preferably a resin having an ester bond such as an acrylic resin or a methacrylic resin, to adsorb xanthophylls, and eluting the xanthophylls with a polar solvent such as an alcohol or acetone. In particular, xanthophylls can be collected efficiently at a high concentration from an oily material obtained from algae or the like in which xanthophylls such as astaxanthins are dissolved at a low concentration. As a result, a concentrate of xanthophylls that contains a xanthophyll fatty acid ester as a main component and has a xanthophyll content of 30 wt% or more in terms of free xanthophyll is provided.

The concentration of the xanthophyll fatty acid ester in this concentrate is estimated to be about 43 wt% or more, and can be about 70 wt% or more. Such a high concentration of xanthophyll fatty acid ester is useful as a material for pigments in food, a fish feed, a raw material for pharmaceuticals and intermediates thereof, and the like.

Best Mode for Carrying Out the Invention

In this specification, "xanthophyll" refers to a compound containing an oxygen atom in the carotene structure, and includes also a compound further having one or two hydroxyl groups in addition to the oxygen atom. Examples of the compound having one hydroxyl group include cryptoxanthin and phenicoxanthin. Examples of the compound having two hydroxyl groups include zeaxanthin, 4-ketozeaxanthin, and astaxanthin.

The method of the present invention for collecting xanthophylls includes a step of contacting a sample containing xanthophylls with a hydrophilic resin. First, A: the sample containing xanthophylls, i.e., a starting material, and B: the hydrophilic resin will be described, and then, C: the method for collecting xanthophylls will be described.

A: Starting Material

The term "xanthophylls" as used in this specification is meant to include at least one xanthophyll selected from the group consisting of a xanthophyll that does not contain a hydroxyl group; a free xanthophyll that has one or two hydroxyl groups; a fatty acid monoester of a xanthophyll, which is in a form in which a fatty acid is bonded to one hydroxyl group; and a fatty acid diester of a xanthophyll, which is in a form in which a fatty acid is bonded to each of two hydroxyl groups. For example, astaxanthin is a xanthophyll having two hydroxyl groups, and thus, the term "astaxanthins" as used herein means at least one compound selected from the group consisting of an astaxanthin (free form), an astaxanthin fatty acid monoester, and an astaxanthin fatty acid diester.

Examples of the fatty acid that can form a fatty acid ester with xanthophylls include lauric acid, myristic acid, pentadecanoic acid, palmitic acid, palmitoleic acid, heptadecanoic acid, elaidic acid, ricinoleic acid, a petroselinic acid, vaccenic acid, eleostearic acid, punicic acid, licanic acid, parinaric acid, gadoleic acid, 5-eicosenoic acid, 5-docosenoic acid, cetoleic acid, erucic acid, 5,13-docosadienoic acid, selacholeic acid, decenoic acid, dodecenoic acid, oleic acid, stearic acid, eicosapentaenoic acid, docosahexaenoic acid, linoleic acid, $\alpha$-linolenic acid, and arachidonic acid.

There is no particular limitation on the source of the xanthophylls used in the method of the present invention are derived. For example, xanthophyll extracts from the fruit, the petals, and the like of plants and xanthophyll extracts from algae can be used, but in the present invention it is not limited to these extracts. In this specification, such an extract is referred to as a "sample containing xanthophylls that is brought into contact with a hydrophilic resin". Alternatively, the sample containing xanthophylls may be in a form in which xanthophylls are dissolved in an oily material such as fats and oils, and in this specification, such a sample is referred to as an "oily material containing xanthophylls". Furthermore, xanthophylls or the above-described sample containing xanthophylls may be dissolved in an organic solvent as described in detail below in the section C-1: Adsorption Step.

The xanthophyll extracts from algae are now described. In algae such as *Haematococcus,* astaxanthins, which are xanthophylls, are accumulated in algal cells, and at the same time, an oily material such as neutral lipids is also produced. In an extraction method using an organic solvent, which is a method commonly employed for extraction of astaxanthins, astaxanthins are extracted together with neutral lipids and the like. The concentration of astaxanthins in an oily material derived from algae is generally about 1 to 10 wt%, although it depends on the extraction solvent, the extraction method, and the like. It should be noted that depending on the culture conditions, astaxanthins from algae contain astaxanthin fatty acid monoesters as main components, and these monoesters constitute at least 50 wt% or more, more preferably 60 wt% or more, even more preferably 70 wt% or more, of total xanthophylls. Astaxanthin fatty acid diesters are present at about 15 to 30 wt%, and the free astaxanthin content is often 1 wt% or less. An astaxanthin-containing oily material derived from algae belonging to *Haematococcus,* a genus of green algae, preferably *Haematococcus pluvialis,* can be used preferably.

B: Hydrophilic Resin

There is no particular limitation on the "hydrophilic resin" used in the present invention, as long as the resin has different adsorption characteristics between xanthophylls, i.e., the target substance, and contaminants, in particular, neutral lipids. Examples of such a resin include a resin that has ester bonds and has backbones made of acrylic acid and/or methacrylic acid and esters thereof. For example, the following resins can be used preferably: a polyacrylic resin;

a polymethacrylic resin; a copolymer resin of acrylate and methacrylate; and a copolymer resin of acrylate and/or methacrylate and methyl methacrylate, polyethyl methacrylate, polymethyl acrylate, polyethyl acrylate, or the like. It is also possible to use methacrylate copolymer resins (product names: HP2MG (made by Mitsubishi Chemical Corporation) and XAD7HP (made by ORGANO CORPORATION)), which are commercially available synthetic adsorbents.

C: Method for Collecting Xanthophylls

The method of the present invention for collecting xanthophyll includes a step of contacting a sample containing xanthophylls with a hydrophilic resin (hereinafter sometimes referred to simply as a "resin") to allow the resin to adsorb the xanthophyll (adsorption step), a step of washing the resin, if necessary, and a step of eluting the xanthophylls adsorbed to the resin with a polar solvent (desorption step).

C-1: Adsorption Step

When xanthophylls are dissolved in an organic solvent, the organic solvent containing xanthophylls may be brought into contact with a resin. Depending on the solvent used, there is a possibility that the xanthophylls may not be adsorbed to the resin. In this case, it is preferable to dilute the organic solvent with a solvent such as alcohols, saturated hydrocarbons, aromatic hydrocarbons, ethers, esters, halogenated hydrocarbons, and ketones or substitute the organic solvent with a solvent as mentioned above, before allowing the resin to adsorb the xanthophylls. As to the alcohols, a lower alcohol having 1 to 5 carbon atoms is preferable, and methanol, ethanol, propanol, and isopropanol are more preferable. Examples of the saturated hydrocarbons include pentane, hexane, heptane, octane, petroleum ether, and kerosene. Examples of the aromatic hydrocarbons include toluene and xylene. Examples of the ethers include diethyl ether and tetrahydrofuran. Examples of the esters include methyl acetate and ethyl acetate. Examples of the halogenated hydrocarbons include dichloroethane and carbon tetrachloride. Examples of the ketones include acetone and methyl ethyl ketone.
When xanthophylls are dissolved in an oily material, the oily material may be brought into contact with a resin to allow the resin to adsorb the xanthophylls. Alternatively, the oily material containing xanthophylls may be preliminarily dissolved in or diluted with the above-described alcohols, saturated hydrocarbons, aromatic hydrocarbons, ethers, esters, halogenated hydrocarbons, or ketones, and then brought into contact with the resin. The above-described organic solvents may be used either alone or in combination. The amount of the solvent, such as an alcohol, that is added to the oily material at the time of adsorption is preferably from an equal amount to a 300-fold amount (volume ratio), more preferably a 2-fold to 100-fold amount, relative to the oily material containing xanthophylls.

C-2: Washing Step

Although it is not essential, the washing step is preferred. The washing step is useful particularly when xanthophylls are dissolved in an oily material. Impurities can be removed by dissolving the oily material, such as neutral lipids, in an organic solvent, such as alcohols, saturated hydrocarbons, aromatic hydrocarbons, ethers, esters, halogenated hydrocarbons, and ketones, used for washing. Examples of the organic solvent used for washing include alcohols, pentane, hexane, heptane, octane, petroleum ether, kerosene, toluene, xylene, diethyl ether, tetrahydrofuran, methyl acetate, ethyl acetate, dichloroethane, carbon tetrachloride, acetone, and methyl ethyl ketone. It should be noted that when a solvent having a high desorption ability such as acetone or methyl ethyl ketone is used, xanthophyll, i.e., the target substance, may be desorbed together with contaminants, and therefore it may be difficult to separate the contaminants from the target substance. For retention of the adsorption of xanthophylls to the adsorption resin, it is preferable to employ alcohols or saturated hydrocarbons. As to the alcohols, methanol, ethanol, propanol, and isopropanol are preferable, and as to the saturated hydrocarbons, pentane, hexane, and heptane can be used preferably. The organic solvents used for washing may be used either alone or in combination. There is no particular limitation on the amount of the organic solvent used for washing. The organic solvent can be used in an equal amount to a 20-fold amount, preferably a 2-fold to 10-fold amount, relative to the volume of the resin used. The solvent used in the washing step may be the same as or different from the solvent used in the adsorption step. For example, when an oily material containing xanthophylls was dissolved in an alcohol or hexane, either an alcohol or hexane may be used in the washing step.

C-3: Desorption Step

The desorption step is a step in which xanthophylls adsorbed to a hydrophilic resin are eluted with a polar solvent. There is no particular limitation on the polar solvent used. For example, alcohols, ethers, esters, halogenated hydrocarbons, and ketones can be used preferably. Examples of the alcohols include methanol, ethanol, propanol, isopropanol, and n-butanol. Examples of the ethers include diethyl ether and tetrahydrofuran. Examples of the esters include methyl acetate and ethyl acetate. Examples of the halogenated hydrocarbons include ethylene dichloride. Examples of the ketones include acetone and methyl ethyl ketone. These organic solvents may be used either alone or in combination. There is no particular limitation on the amount of the polar organic solvent used for desorption. The polar organic solvent can be used in from an equal amount to a 50-fold amount, preferably a 2-fold to 10-fold amount, relative to the volume of the resin used.

C-4: Adsorption-Washing-Desorption Steps

There is no particular limitation on the method for allowing a resin to adsorb xanthophylls. The sequence adsorption-washing-desorption may be performed in a batchwise operation while stirring, or may be performed using a column packed with the resin. From the point of view of process control, it is preferable to perform these steps using a column.

C-5: Combination of Organic Solvents Used in the Respective Steps

The organic solvents used in the respective steps may be the same in all of the steps, or may be different from one another. For example, all of the solvents used in the sequence adsorption step-washing step-desorption step may be ethanol, or the solvents may be respectively methanol-ethanol-ethanol.

However, by using the same solvent, e.g., ethanol, in the respective steps, it also is possible to collect a high concentration of xanthophylls with a single solvent. As a result, it is easier to recover the solvent, so that the solvent can be recycled. Furthermore, when a concentrate containing xanthophylls is used for food products, pharmaceuticals, and the like, it is required that the concentrate does not contain a toxic chemical. However, even when the concentrate contains residual ethanol, ethanol has very low toxicity and thus does not cause a problem. As described above, it is very advantageous to use ethanol in all of the steps in terms of production of xanthophylls and the quality of resultant xanthophylls.

On the other hand, for example, when a methacrylate copolymer resin is employed and ethanol is used in the washing step, xanthophylls, i.e., the target substance, may be desorbed gradually in this washing step. Accordingly, the concentration and the collection rate of xanthophylls vary significantly depending on the point in time when collection of the eluate is started. Therefore, when such a solvent is selected, the point in time when collection is started should be determined by considering which of the concentration or the collection rate has the higher priority.

D: Concentrate of Xanthophyll

According to the above-described method, a concentrate of xanthophylls is produced. This concentrate contains xanthophylls comprising xanthophyll fatty acid esters as main components and having a xanthophyll content of 30 wt% or more in terms of free xanthophyll.

When the xanthophyll content is 30 wt%, the concentration of xanthophyll fatty acid esters is estimated at about 45 wt%. In the case of a concentrate produced using green algae, e.g., algae from the genus *Haematococcus,* most of the xanthophyll is astaxanthin. From an analysis of a commercially available astaxanthin-containing product derived from *Haematococcus,* it is believed that the average molecular weight of fatty acids bonded to astaxanthin is about 290 and that the percentage of monoesters is 75% and that of diesters is 25%. Therefore, according to the method of the present invention, when a concentrate having an astaxanthin content of 30 wt% or more in terms of free astaxanthin is obtained, astaxanthin fatty acid esters can be collected at an astaxanthin fatty acid ester concentration of at least about 43 wt%.

Examples

Hereinafter, the present invention will be described by way of examples. However, the present invention is not limited to these examples. In the examples, a *Haematococcus* extract obtained from green algae, *Haematococcus pluvialis,* was used as an example of the oily material containing xanthophylls. Most of the xanthophylls in this *Haematococcus* extract are astaxanthins. This *Haematococcus* extract containing xanthophylls is available from the Japan Food Additives Association as a food additive *"Haematococcus* algae pigment".

The xanthophyll concentration (the concentration of xanthophylls in terms of free xanthophyll) was calculated using the following equation:

$$\text{Total xanthophyll concentration (wt\%)} = (A * 100 * F)/(W * 2085)$$

where

A is the absorbance at 478 nm of a sample (optical path length 1 cm),
F is the dilution factor of the sample, and
W is the weight (g) of the sample.

The composition of xanthophylls was analyzed by high-performance liquid chromatography.

Example 1

A column having a diameter of 20 mm and packed with 30 ml of a methacrylate copolymer resin (product name: HP2MG, made by Mitsubishi Chemical Corporation) was prepared. Then, 201 mg of a *Haematococcus* extract containing xanthophylls (xanthophyll concentration 9.6 wt%) dissolved in ethanol (12 ml) was passed through this column to adsorb the xanthophylls. Next, 60 ml of ethanol were passed through this column to wash the resin. This fraction eluted by washing was collected, developed by thin layer chromatography (TLC), and analyzed by color development using a phosphomolybdic acid method. When the fraction was developed using a developing solvent (petroleum ether:acetone (4:1, volume ratio)), fats and oils containing triacylglycerides (TG) as main components and a small amount of xanthophylls were detected. After washing with ethanol, an additional 160 ml of ethanol was passed through the column at SV = 1.5 to collect the xanthophylls. At the time of collection, the eluate was collected in fractions of 10 ml each. Each fraction was diluted 10-fold with an eluent (ethanol), and the absorbance at 478 nm was measured. The fractions having an absorbance of 1.0 or more and in which almost no substance other than the xanthophylls was detected by observation using TLC were combined, and ethanol was removed by distillation to give 19.8 mg of a concentrate containing 43 wt% of xanthophyll. Thus, the xanthophylls were concentrated by a factor of about 4.5, and the recovery of the xanthophylls was 44%. A combination of the solvent used in the sequence adsorption step- washing step- desorption step in Example 1 was ethanol-ethanol-ethanol. These results are shown in Table 1 all together.

Example 2

A concentrate of xanthophyll was obtained in the same manner as in Example 1, except that 196 mg of a *Haematococcus* extract (xanthophyll concentration 9.6 wt%) were used; the solvents used in the sequence adsorption step- washing step- desorption step were respectively ethanol-ethanol-acetone; and the amount of acetone used was 60 ml. The results are shown in Table 1.

Example 3

A concentrate of xanthophyll was obtained in the same manner as in Example 1, except that 205 mg of a *Haematococcus* extract (xanthophyll concentration 9.6 wt%) were used; the solvents used in the sequence adsorption step- washing step- desorption step were respectively hexane-hexane-ethanol; and the amount of hexane used in the washing step was 165 ml. The results are shown in Table 1. In Example 3, the oily component comprising TG was not sufficiently washed out with hexane, and the residual oily component was eluted with ethanol as the desorption solvent. In a TLC analysis, it was observed that when ethanol was passed through the column, the oily component tended to be eluted first, followed by xanthophylls.

Example 4

A concentrate of xanthophyll was obtained in the same manner as in Example 3, except that 211 mg of a *Haematococcus* extract (xanthophyll concentration 9.6 wt%) were used and the solvents used in the sequence adsorption step- washing step- desorption step were respectively hexane-hexane-acetone. The results are shown in Table 1. In Example 4, the oily component comprising TG was not sufficiently washed out with hexane, and the residual oily component was eluted with acetone used as the desorption solvent. In a TLC analysis, it was observed that when acetone was passed through the column, the oily component tended to be eluted first, followed by xanthophylls.

Example 5

A concentrate of xanthophyll was obtained in the same manner as in Example 1, except that 207 mg of a *Haematococcus* extract (xanthophyll concentration 9.6 wt%) were used and the solvents used in the sequence adsorption step- washing step- desorption step were respectively hexane-ethanol-ethanol. The results are shown in Table 1.

Example 6

A concentrate of xanthophyll was obtained in the same manner as in Example 4, except that 199 mg of a *Haematococcus* extract (xanthophyll concentration 9.6 wt%) were used; the solvents used in the sequence adsorption step- washing step- desorption step were respectively hexane-ethanol-acetone; and the amount of ethanol used was 60 ml. The results are shown in Table 1. In Example 6, the oily component comprising TG was almost completely washed out with ethanol, and elution of xanthophylls in the washing step was hardly observed.

Example 7

A concentrate of xanthophyll was obtained in the same manner as in Example 2, except that 206 mg of a *Haematococcus* extract (xanthophyll concentration 9.6 wt%) were used and a methacrylate copolymer resin (product name: XAD7HP, made by ORGANO CORPORATION) was used. The results are shown in Table 1.

Comparative Example 1

A concentrate of xanthophyll was obtained in the same manner as in Example 1, except that 202 mg of a *Haematococcus* extract (xanthophyll concentration 9.6 wt%) were used and the solvents used in the sequence adsorption step- washing step- desorption step were respectively ethanol-ethanol-hexane. The results are shown in Table 2.

Comparative Example 2

A concentrate of xanthophyll was obtained in the same manner as in Example 2, i.e., using ethanol-ethanol-acetone as the respective solvents in the sequence adsorption step- washing step- desorption step, except that 200 mg of a *Haematococcus* extract (xanthophyll concentration 11 wt%) were used and a styrene resin (product name: HP20, made by Mitsubishi Chemical Corporation) was used. The results are shown in Table 2.

Comparative Example 3

A concentrate of xanthophyll was obtained in the same manner as in Comparative Example 2, except that 189 mg of a *Haematococcus* extract (xanthophyll concentration 11 wt%) were used and a modified styrene resin (product name: SP207, made by Mitsubishi Chemical Corporation) was used. The results are shown in Table 2.

Comparative Example 4

A concentrate of xanthophyll was obtained in the same manner as in Comparative Example 2, except that 210 mg of a *Haematococcus* extract (xanthophyll concentration 11 wt%) were used and a phenolic resin (product name: XAD761, made by ORGANO CORPORATION) was used. The results are shown in Table 2.

## Table 1

| | | | Raw material | Example | | | | | | |
|---|---|---|---|---|---|---|---|---|---|---|
| | | | | 1 | 2 | 3 | 4 | 5 | 6 | 7 |
| Step | Resin used | Name | | HP2MG | HP2MG | HP2MG | HP2MG | HP2MG | HP2MG | XAD7HP |
| | | Type | | Methacrylate | Methacrylate | Methacrylate | Methacrylate | Methacrylate | Methacrylate | Acrylate |
| | Absorption solvent | | - | Ethanol | Ethanol | Hexane | Hexane | Hexane | Hexane | Ethanol |
| | Washing solvent | | - | Ethanol | Ethanol | Hexane | Hexane | Ethanol | Ethanol | Ethanol |
| | Desorption solvent | | - | Ethanol | Acetone | Ethanol | Acetone | Ethanol | Acetone | Acetone |
| Xanthophyll | Xanthophyll concentration factor (fold factor) | | - | 4.5 | 4.6 | 3.4 | 3.3 | 5 | 4.8 | 4.5 |
| | Concentration of xanthophyll[1] | | - | 43 | 44 | 33 | 32 | 48 | 46 | 43 |
| | Recovery of xanthophyll (%) | | - | 44 | 47 | 86 | 97 | 59 | 64 | 45 |
| | Composition | Free astaxanthin (%)[2] | 0.5 | 0.5 | 0.5 | 0.6 | 0.5 | 0.2 | 0.5 | 0.6 |
| | | Astaxanthin monoester (%) | 76.8 | 76 | 74.9 | 79.4 | 75.2 | 66.1 | 81.2 | 76.6 |
| | | Astaxanthin diester (%) | 20.9 | 22.5 | 23.4 | 18.7 | 22.6 | 31.6 | 17 | 21.4 |
| | | Canthaxanthin (%) | 0.1 | 0.1 | 0.1 | 0.2 | 0.1 | 0 | 0.1 | 0.2 |
| | | Zeaxanthin + Lutein (%) | 0.6 | 0 | 0 | 0.3 | 0.6 | 1.2 | 1 | 0.1 |
| | | Adonirubin ester (%) | 1 | 0.9 | 1.1 | 0.9 | 0.9 | 0.9 | 0.1 | 1 |

*1: expressed in terms of free xanthophyll

*2: weight (%)

Table 2

| | | Raw material | Comparative Example | | | |
|---|---|---|---|---|---|---|
| | | | 1 | 2 | 3 | 4 |
| Step | Resin used — Name | - | HP2MG | SP207 | SP207 | XAD761 |
| | Resin used — Type | - | Methacrylate | Styrene | Modified styrene | Phenol |
| | Absorption solvent | - | Ethanol | Ethanol | Ethanol | Ethanol |
| | Washing solvent | - | Ethanol | Ethanol | Ethanol | Ethanol |
| | Desorption solvent | - | Hexane | Acetone | Acetone | Acetone |
| | Xanthophyll concentration factor (fold factor) | - | 3.8 | 1 | 0.9 | 0.9 |
| | Concentration of xanthophyll[*1] | - | 36 | 11 | 10 | 10 |
| | Recovery of xanthophyll (%) | - | 16 | 96 | 86 | 86 |
| Xanthophyll — Composition | Free astaxanthin (%)[*2] | 0.5 | 0.8 | 0.5 | 0.6 | 0.5 |
| | Astaxanthin monoester (%) | 76.8 | 72.1 | 77.4 | 81.8 | 76.6 |
| | Astaxanthin diester (%) | 20.9 | 27 | 20.5 | 16.5 | 21.1 |
| | Canthaxanthin (%) | 0.1 | 0.2 | 0.1 | 0.1 | 0.1 |
| | Zeaxanthin + Lutein (%) | 0.6 | 0.1 | 0.5 | 0.5 | 0.6 |
| | Adonirubin ester (%) | 1 | 0.3 | 0.9 | 0.5 | 0.9 |

*1: expressed in terms of free xanthophyll
*2: weight (%)

As can be seen from Tables 1 and 2, when a hydrophilic resin such as a methacrylate copolymer resin was used and a polar solvent such as ethanol or acetone was used as the eluent in the desorption step, xanthophylls were concentrated by a factor of 3.3 or more, preferably 4 or more, and also the concentration of xanthophyll in terms of free xanthophyll was high. The concentration of xanthophyll in terms of fatty acid ester is about 1.5 times higher than that of the free xanthophyll (wt%).

On the other hand, it was found that as in Comparative Example 1, when hexane, which is hydrophobic, was used as the eluent, the recovery of xanthophylls was very poor although the concentration factor was increased. Moreover, in Comparative Examples 2 to 4 in which the hydrophobic styrene resins are used for purification of carotene, the recovery of xanthophylls was high, but the concentration of xanthophyll was hardly improved and purification could not be achieved.

Comparative Example 5

An attempt to concentrate xanthophylls was made in the same manner as in Example 1, except that 196 mg of a *Haematococcus* extract (xanthophyll concentration 9.6 wt%) were used and the solvents used in the sequence adsorption step- washing step- desorption step were respectively hexane-hexane-hexane. However, xanthophylls, i.e., the target substance, remained adsorbed to the resin, and a concentrate containing xanthophylls was not obtained (data is not shown in tables).

Industrial Applicability

The method of the present invention is a superior method for collecting xanthophylls, in particular, xanthophylls dissolved in an oily material, and is very useful in production of xanthophylls because xanthophylls can be concentrated to a high concentration by simply using a combination of a hydrophilic resin and a single solvent having low toxicity. Moreover, the concentrate provided by the method of the present invention wherein xanthophylls bonded to a fatty acid is highly stable and safe, and also contains the xanthophylls at a high concentration, so that the concentrate is useful for production of food products, pharmaceuticals, and the like.

**Claims**

1. A method for collecting xanthophylls, comprising:

    an adsorption step of contacting a sample containing the xanthophylls with a hydrophilic resin to allow the resin to adsorb the xanthophylls; and
    a desorption step of eluting the xanthophylls adsorbed to the resin with a polar solvent.

2. The method of claim 1, further comprising a washing step of washing the hydrophilic resin between the adsorption step and the desorption step.

3. The method of claim 1 or 2, wherein the hydrophilic resin is an acrylic and/or methacrylic resin.

4. The method of any one of claims 1 to 3, wherein the sample containing xanthophylls that is brought into contact with the hydrophilic resin is an oily material containing xanthophylls.

5. The method of claim 4, wherein the oily material containing xanthophylls is obtained from an alga.

6. The method of claim 5, wherein the alga is a green alga belonging to the genus *Haematococcus.*

7. The method of any one of claims 1 to 6, wherein the sample containing xanthophylls that is brought into contact with the hydrophilic resin is dissolved in at least one solvent selected from the group consisting of alcohols, saturated hydrocarbons, aromatic hydrocarbons, ethers, esters, halogenated hydrocarbons, and ketones.

8. The method of claim 7, wherein the alcohols, saturated hydrocarbons, aromatic hydrocarbons, ethers, esters, halogenated hydrocarbons, and ketones are at least one solvent selected from the group consisting of methanol, ethanol, propanol, isopropanol, pentane, hexane, heptane, toluene, xylene, tetrahydrofuran, diethyl ether, methyl acetate, ethyl acetate, dichloromethane, carbon tetrachloride, acetone, and methyl ethyl ketone.

9. The method of any one of claims 1 to 8, wherein the polar solvent used to elute the xanthophylls is at least one solvent selected from the group consisting of methanol, ethanol, propanol, isopropanol, n-butanol, tetrahydrofuran, diethyl ether, methyl acetate, ethyl acetate, acetone, and methyl ethyl ketone.

10. The method of any one of claims 1 to 9, wherein the xanthophylls are astaxanthins.

11. A concentrate of xanthophylls comprising xanthophylls, wherein the xanthophylls contain a xanthophyll fatty acid ester as a main component and have a xanthophyll content of 30 wt% or more in terms of free xanthophyll.

12. The concentrate of claim 11, wherein the concentrate is obtained by a method comprising:

contacting a sample containing xanthophylls with a hydrophilic resin to allow the hydrophilic resin to adsorb the xanthophylls; and
eluting the xanthophylls adsorbed to the hydrophilic resin with a polar solvent.

13. The concentrate of claim 11 or 12, wherein the concentrate of xanthophylls is a concentrate of astaxanthins.

14. The concentrate of claim 13, wherein the astaxanthins are obtained from a green alga belonging to the genus *Haematococcus.*

## INTERNATIONAL SEARCH REPORT

| | International application No. |
|---|---|
| | PCT/JP2006/303537 |

**A. CLASSIFICATION OF SUBJECT MATTER**
*C07C403/24*(2006.01), *A23L1/27*(2006.01)

According to International Patent Classification (IPC) or to both national classification and IPC

**B. FIELDS SEARCHED**

Minimum documentation searched (classification system followed by classification symbols)
C07C403/24, A23L1/27

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-41147 A  (Takeda Shiki Kabushiki Kaisha),<br>12 February, 2004 (12.02.04),<br>Claims; Par. No. [0026]; examples<br>(Family: none) | 11<br>5-14 |
| X<br>Y | JP 2002-255931 A  (BASF AG.),<br>11 September, 2002 (11.09.02),<br>Examples<br>& EP 1213013 A2        & US 2002/110599 A1 | 11<br>11-14 |
| X<br>Y | JP 7-265088 A  (Marine Biotechnology Institute<br>Co., Ltd.),<br>17 October, 1995 (17.10.95),<br>Full text (particularly, Par. No. [0012])<br>(Family: none) | 1,4,7-9<br>1-14 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 March, 2006 (24.03.06) | 04 April, 2006 (04.04.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2006/303537 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 63-5073 A (Lion Corp.),<br>11 January, 1988 (11.01.88),<br>Full text<br>(Family: none) | 1-14 |
| Y | JP 2002-218994 A (Fuji Chemical Industry Co.,<br>Ltd.),<br>06 August, 2002 (06.08.02),<br>Full text<br>& WO 2002/059341 A1 & EP 1361281 A1<br>& US 2004/115758 A1 | 5-10,13,14 |
| A | JP 61-12657 A (Lion Corp.),<br>21 January, 1996 (21.01.86),<br>& JP 61-115062 A & GB 2160874 A | 1-14 |
| A | JP 61-282357 A (Director General, Agency of<br>Industrial Science and Technology),<br>12 December, 1986 (12.12.86),<br>(Family: none) | 1-14 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3266719 B
- WO 11508603 A
- JP 2004041147 A
- JP 61012657 A
- JP 63005073 A

**Non-patent literature cited in the description**

- **C. SATO.** *New Food Industry,* 2002, vol. 44 (10), 1-5
- **K. YAZAWA.** *New Food Industry,* 2004, vol. 46 (5), 17-24
- **MASAYOSHI KAZAMA et al.** *The Food Industry,* 2003, vol. 46 (24), 25-35
- **PHYLIPS E. BROWEN et al.** *J. Nutr.,* 2002, vol. 132, 3668-3673
- **B. S. BAHARIN et al.** *JAOCS,* 1998, vol. 75 (3), 399-404
- **R. A. LATIP et al.** *JAOCS,* 2000, vol. 77 (12), 1277-1281
- **K. W. CHAN et al.** *J. Food Lipids,* 2000, vol. 7, 127-141